# EUROPEAN PATENT SPECIFICATION

(11) **EP 3 300 662 B1**
(45) Date of publication and mention of the grant of the patent: **17.08.2022**
(21) Application number: 16191688.7
(22) Date of filing: 30.09.2016
(51) Int. Cl.: A61B 5/05, A61B 5/11, A61B 5/00, A61D 17/00, A01K 29/00, A61B 5/16

(54) **DETERMINING AN INTIMATE ACTIVITY BY A DETECTION DEVICE**
BESTIMMUNG EINER INTIMEN AKTIVITÄT DURCH EINE DETEKTIONSVORRICHTUNG
DÉTERMINATION D'UNE ACTIVITÉ INTIME PAR UN DISPOSITIF DE DÉTECTION

(43) Date of publication of application: 04.04.2018
(73) Proprietor: Nokia Technologies Oy, 02610 Espoo (FI)
(72) Inventor: BAILEY, Marc, Cambridge, CB4 3DD (GB); SHAMAIN, Durgaprasad, San Jose, CA 95154 (US); MUNIRAJU, Swetha, Sunnyvale, CA 94086 (US)
(74) Representative: Whiting, Gary

(56) References cited:
- WO-A1-2015/056300
- WO-A2-2015/102713
- US-A1- 2009 234 200
- US-A1- 2013 300 573
- US-A1- 2014 058 255

## Description

### TECHNOLOGICAL FIELD

An example embodiment of the present disclosure relates generally to interpretation of data collected by a detection device. More particularly in some cases, to a method, apparatus and computer program product for determining an intimate activity has occurred based on data collected with a radar detection device.

### BACKGROUND

In the area of sleep research and sleep behavior, scientists and medical professionals may use radar detection devices to detect and monitor the movements, positioning, and behavior of people in bed. Researchers and sleep study applications may process data captured by a bedside radar detection device, for example, and analyze various cycles of sleep based on tossing and turning, rolling, and the like. The radar systems used in such studies can detect subtle movements or changes in positioning, and use the data to gather information about the user's sleep habits, patterns, and effect of everyday activities on one's sleep. Nevertheless, it can be challenging to discriminate between various types of activities of the studied participants, such as when the participants are sleeping or are awake, as desired for an effective sleep study.

Document US 2013/0300573 A1 discloses a radar system for remote monitoring of a patient. Document WO 2015/056300 A1 discloses a patient monitoring system comprising a privacy protection mode.

Document US 2009/0234200 A1 discloses a temperature tracking system which ignores overnight temperature measurements taken during periods of high movement of the subject.

### BRIEF SUMMARY

A method, apparatus, and computer program product according to the present invention, defined in claims 1, 11 and 13, are therefore provided for determining an intimate activity has occurred based on data collected with a radar detection system. Example embodiments may process and determine baseline positioning information of individuals relative to a bed, as captured by a radar detection system. For example, the baseline may be considered the first position of the users upon entering the bed, or an average or standard position of the participants during ordinary sleep. The radar detection device may then gather subsequent data relating to the positioning and movements occurring in the bed. Based on the changes of the positioning and/or movements of users, example embodiments may identify occurrences of intimate activity. The determined intimate activity may include any physical activity occurring between at least two individuals that is not attributed to sleep, such as but not limited to, a sexual activity. In some cases, the occurrences of intimate activity may be obscured or deleted from a data record, for example, to maintain the privacy of the users. However, in some cases, an occurrence of intimate activity detected by the innovation described herein may be used via analysis of data associated with the occurrence, obscured by assignment of metadata associated with the occurrence, or any combination thereof.

A method is provided for receiving a baseline position indication according to a radar signal detected from a radar detection device and relating to a plurality of body profiles. The baseline position indication comprises baseline position information of each body profile relative to each other and a common reference point. The method also comprises receiving an updated position indication detected from the radar detection device and relating to the plurality of body profiles. The updated position indication comprises updated position information of each body profile relative to each other and the common reference point. The method further comprises determining, with a processor, that a change from the baseline position indication to the updated position indication is indicative of intimate activity.

In some examples, the method further comprises determining an end to the intimate activity based on a subsequent radar signal, and/or determining temporal indicators of a start and end of the intimate activity. According to the invention, the method further comprises compiling a sleep data record representative of sleep patterns associated with the plurality of body profiles; and excluding a portion of data corresponding to the intimate activity from the sleep data record. In some examples, the portion of data corresponding to the intimate activity is excluded from the sleep data record in response to a user input.

In some embodiments, the change indicative of the plurality of body profiles engaging in intimate activity is based on at least a change in distance of at least one of the plurality of body profiles from the radar detection device. In some examples, the change indicative of the plurality of body profiles engaging in intimate activity is based on a change in at least a height or width of a cross-section of the plurality of body profiles. In some embodiments, the change indicative of the plurality of body profiles engaging in intimate activity is based on a variation of the detected radar signal as a function of time, an intensity of the detected radar signal, a frequency of the detected radar signal, and/or a change in distance of the plurality of body profiles from each other.

An apparatus is provided in accordance with another example embodiment that comprises at least one processor and at least one memory including computer program code with the at least one memory and the computer program code configured to, with the processor, cause the apparatus to at least receive a baseline position indication according to a radar signal detected from a radar detection device and relating to a plurality of body profiles. The baseline position indication comprises baseline position information of each body profile relative to each other and a common reference point. The at least one memory and the computer program code are also configured to, with the processor, cause the apparatus to receive an updated position indication detected from the radar detection device and relating to the plurality of body profiles. The updated position indication comprises updated position information of each body profile relative to each other and the common reference point. The at least one memory and the computer program code are also configured to, with the processor, cause the apparatus to determine that a change from the baseline position indication to the updated position indication is indicative of the plurality of body profiles engaging in intimate activity. The at least one memory and the computer program code are further configured to cause the apparatus to perform any of the methods described herein.

An apparatus is provided that comprises at least one signal detection device, at least one processor, and at least one memory including computer program code. The at least one memory and the computer program code configured to, with the processor, cause the apparatus to at least generate a baseline position indication according to a signal detected from the signal detection device and relating to a plurality of body profiles, wherein the baseline position indication comprises baseline position information of each body profile relative to each other and a common reference point. The at least one memory and the computer program code are also configured to, with the processor, cause the apparatus to generate an updated position indication according to the signal detected from the signal detection device and relating to the plurality of body profiles. The updated position indication comprises updated position information of each body profile relative to each other and the common reference point. The at least one memory and the computer program code are also configured to, with the processor, cause the apparatus to determine that a change from the baseline position indication to the updated position indication is indicative of the plurality of body profiles engaging in intimate activity. In some examples, the signal detection device comprises a radar detection device configured to emit a radar signal with a transmitter, and with a transceiver, detect the radar signal reflected from the plurality of body profiles and the common reference point, wherein the baseline position indication and the updated position indication are generated according to the reflected radar signal.

A computer program product is provided that comprises at least one non-transitory computer-readable storage medium having computer-executable program code instructions stored therein with the computer-executable program code instructions comprising program code instructions to at least receive a baseline position indication according to a radar signal detected from a radar detection device and relating to a plurality of body profiles. The baseline position indication comprises baseline position information of each body profile relative to each other and a common reference point. The computer-executable program code instructions also comprise program code instructions to receive an updated position indication detected from the radar detection device and relating to the plurality of body profiles. The updated position indication comprises updated position information of each body profile relative to each other and the common reference point. The computer-executable program code instructions further comprise program code instructions to determine that a change from the baseline position indication to the updated position indication is indicative of the plurality of body profiles engaging in intimate activity. The computer-executable program code instructions further comprise program code instructions to perform any of the methods described herein.

An apparatus is provided in accordance with yet another example embodiment that comprises means for receiving a baseline position indication according to a radar signal detected from a radar detection device and relating to a plurality of body profiles. The baseline position indication comprises baseline position information of each body profile relative to each other and a common reference point. The apparatus also comprises means for receiving an updated position indication detected from the radar detection device and relating to the plurality of body profiles. The updated position indication comprises updated position information of each body profile relative to each other and the common reference point. The apparatus further comprises means for determining that a change from the baseline position indication to the updated position indication is indicative of intimate activity.

### BRIEF DESCRIPTION OF THE DRAWINGS

Having thus described certain example embodiments of the present disclosure in general terms, reference will hereinafter be made to the accompanying drawings which are not necessarily drawn to scale, and wherein:
Figure 1A is a block diagram of an apparatus that may be configured to implement example embodiments of the present disclosure;
Figure 1B illustrates a radar detection device situated proximate a common reference point;
Figure 2 is a flowchart illustrating operations performed in accordance with example embodiments of the present disclosure;
Figure 3A is an example cross-section view representative of a baseline position indication relating to two body profiles according to example embodiments;
Figure 3B is a plot of detected radar signals representative of the body profiles of Figure 3A according to example embodiments;
Figure 4A is an example cross-section view representative of an updated position indication relating to two body profiles according to example embodiments;
Figure 4B is a plot of detected radar signals representative of the body profiles of Figure 4A according to example embodiments; and
Figure 5 is a plot for plotting detected radar signals representative of body profiles according to example embodiments.

### DETAILED DESCRIPTION

Some embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present disclosure. Thus, use of any such terms should not be taken to limit the scope of embodiments of the present disclosure.

Additionally, as used herein, the term 'circuitry' refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit or applications processor integrated circuit for a mobile phone or a similar integrated circuit in a server, a cellular network device, other network device, field programmable gate array, and/or other computing device.

As defined herein, a "computer-readable storage medium," which refers to a physical storage medium (e.g., volatile or non-volatile memory device), may be differentiated from a "computer-readable transmission medium," which refers to an electromagnetic signal.

As described below, a method, apparatus and computer program product are provided for determining an intimate activity has occurred based on data collected with a signal detection device, such as a radar detection device. The data collected by the radar detection device may be collected in the vicinity of the participants' bed for the purpose of studying sleep record data, but it will be appreciated that the data may be collected and interpreted by any type of application in which the determination of the occurrence of an intimate activity based on the detected radar signals may be of value according to example embodiments. Although a radar detection device is discussed herein, any detection device having elements configured to detect intimate behavior or activity using the techniques described herein may be implemented to carry out the techniques described herein. The techniques described herein may provide a technical effect of detecting certain behaviors of a user that may increase the effectiveness of activity monitoring in fields such as digital health. Other technical effects are discussed herein.

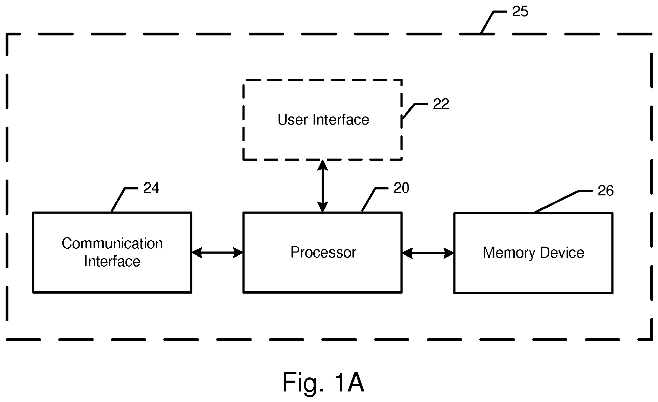

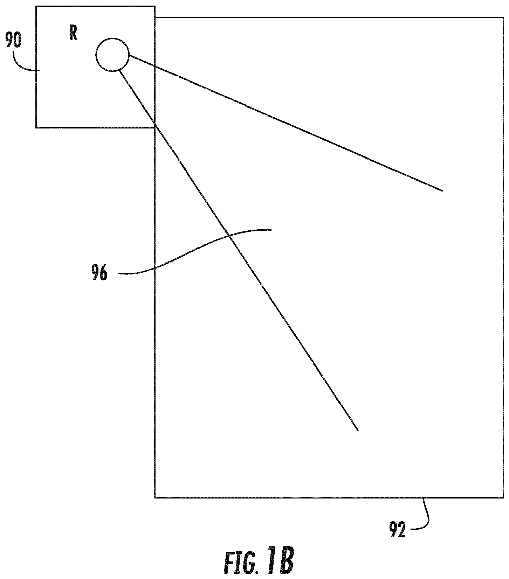

Referring to Figure 1A, apparatus 25 may include or otherwise be in communication with a processor 20, communication interface 24, and memory device 26. As described below and as indicated by the dashed lines in Figure 1A, in some embodiments, the apparatus 25 may also optionally include a user interface 22. Apparatus 25 may be implemented as a server or distributed system, such as a server for processing sleep data, data collected by a radar detection device and/or the like. In some examples, apparatus 25 need not necessarily be embodied by a server, and may be embodied by a wide variety of devices including personal computers, work stations, or mobile terminals, such as laptop computers, tablet computers, smartphones or any combination of the aforementioned, and other types of voice and text communications systems. In some examples, apparatus 25 may be embodied by or otherwise associated with, e.g., in communication with, a radar detection device that emits and detects radar signals for subsequent processing. Positioning and use of the radar detection device is described below with respect to Figure 1B.

In some embodiments, the processor 20 (and/or co-processors or any other processing circuitry assisting or otherwise associated with the processor 20) may be in communication with the memory device 26 via a bus for passing information among components of the apparatus 25. The memory device 26 may include, for example, one or more volatile and/or non-volatile memories. In other words, for example, the memory device 26 may be an electronic storage device (e.g., a computer readable storage medium) comprising gates configured to store data (e.g., bits) that may be retrievable by a machine (e.g., a computing device like the processor 20). The memory device 26 may be configured to store information, data, content, applications, instructions, or the like for enabling the apparatus to carry out various functions in accordance with an example embodiment of the present disclosure. For example, the memory device 26 could be configured to buffer input data for processing by the processor 20. Additionally or alternatively, the memory device 26 could be configured to store instructions for execution by the processor 20. In some embodiments, the memory device 26 may be configured to store sleep data and/or other data collected and/or converted by a radar detection device.

The apparatus 25 may, in some embodiments, be embodied in various devices as described above (e.g., server, work station, and/or the like). In some embodiments, apparatus 25 may embody the signal detect device (e.g., radar detection device), and the processor 20 may be further configured to implement the functionality described herein in addition to the functions of the signal detection device. However, in some embodiments, the apparatus 25 may be embodied as a chip or chip set. In other words, the apparatus 25 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The apparatus 25 may therefore, in some cases, be configured to implement an embodiment of the present disclosure on a single chip or as a single "system on a chip." As such, in some cases, a chip or chipset may constitute means for performing one or more operations for providing the functionalities described herein.

The processor 20 may be embodied in a number of different ways. For example, the processor 20 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor 20 may include one or more processing cores configured to perform independently. A multi-core processor may enable multiprocessing within a single physical package. Additionally or alternatively, the processor 20 may include one or more processors configured in tandem via the bus to enable independent execution of instructions, pipelining and/or multithreading.

In an example embodiment, the processor 20 may be configured to execute instructions stored in the memory device 26 or otherwise accessible to the processor 20. Alternatively or additionally, the processor 20 may be configured to execute hard coded functionality. As such, whether configured by hardware or software methods, or by a combination thereof, the processor 20 may represent an entity (e.g., physically embodied in circuitry) capable of performing operations according to an embodiment of the present disclosure while configured accordingly. Thus, for example, when the processor 20 is embodied as an ASIC, FPGA or the like, the processor 20 may be specifically configured hardware for conducting the operations described herein. Alternatively, as another example, when the processor 20 is embodied as an executor of software instructions, the instructions may specifically configure the processor 20 to perform the algorithms and/or operations described herein when the instructions are executed. However, in some cases, the processor 20 may be a processor of a specific device (e.g., a mobile terminal or network entity) configured to employ an embodiment of the present disclosure by further configuration of the processor 20 by instructions for performing the algorithms and/or operations described herein. The processor 20 may include, among other things, a clock, an arithmetic logic unit (ALU) and logic gates configured to support operation of the processor 20.

Meanwhile, the communication interface 24 may be any means such as a device or circuitry embodied in either hardware or a combination of hardware and software that is configured to receive and/or transmit data from/to a network and/or any other device or module in communication with the apparatus 25. In this regard, the communication interface 24 may include, for example, an antenna (or multiple antennas) and supporting hardware and/or software for enabling communications with a wireless communication network. For example, the communication interface 24 may include an antenna configured to emit or receive radar signals. Additionally or alternatively, the communication interface 24 may include the circuitry for interacting with the antenna(s) to cause transmission of signals via the antenna(s) or to handle receipt of signals received via the antenna(s). In some environments, the communication interface 24 may alternatively or also support wired communication. As such, for example, the communication interface 24 may include a communication modem and/or other hardware/software for supporting communication via cable, digital subscriber line (DSL), universal serial bus (USB) or other mechanisms.

In some embodiments, such as instances in which the apparatus 25 is embodied by a user device, the apparatus 25 may include a user interface 22 that may, in turn, be in communication with the processor 20 to receive an indication of a user input and/or to cause provision of an audible, visual, mechanical or other output to the user. As such, the user interface 22 may include, for example, a keyboard, a mouse, a joystick, a display, a touch screen(s), touch areas, soft keys, a microphone, a speaker, or other input/output mechanisms. Alternatively or additionally, the processor 20 may comprise user interface circuitry configured to control at least some functions of one or more user interface elements such as, for example, a speaker, ringer, microphone, display, and/or the like. The processor 20 and/or user interface circuitry comprising the processor 20 may be configured to control one or more functions of one or more user interface elements through computer program instructions (e.g., software and/or firmware) stored on a memory accessible to the processor 20 (e.g., memory device 26, and/or the like). In some embodiments, the user interface 22 may be configured for a user to confirm that the data processed by apparatus 25 is indeed attributed to an intimate activity, and may be further configured such that the user may indicate to exclude or obscure data associated with an occurrence of the identified intimate activity from being transmitted or stored along with the sleep data record.

Figure 1B illustrates an example radar detection device 90 situated proximate a common reference point 92, which in this example, may be a plane associated with a bed or mattress. The radar detection device 90 used to collect the data processed by apparatus 25 according to example embodiments may be situated in close proximity to the bed, or other common reference point 92 to which relative body profile positions are determined. For example, radar detection device 90 may be placed on a bed side table. For simplicity, the example of a bed or mattress may be referred to hereinafter, but it will be appreciated that any object, such as a couch, floor, or other surface may be used as a reference point from which the radar detection device 90 detects people, or body profiles, and the positioning or movement thereof. In this regard, the reference point 92 may include an (x,y,z) coordinate in space, or may include a top facing planar surface of a mattress, floor, table, couch, for example and/or the like. The "field of view" of the radar detection device 90 may therefore include elements of reflectance from the mattress, bedframe and other furniture which may not vary as long as the positions of the furniture and radar remain fixed.

The radar detection device 90 may be any type of radar detecting unit or system that scans an area proximate the reference point. As another example, the radar detection device 90 may be built within a bed, such as in the box frame or structure of the bed. In some examples, the radar detection device 90 may be implemented within a mobile device, and/or wearable device. The radar detection device 90 may have a beam that is sufficiently wide so as to cover the entire bed or other area of interest. Alternatively, the radar detection device 90 may sweep a radar beam (e.g., radio waves) through an area covering the reference point, or in a general area of the reference point, and may send and receive radar signals. See, for example radar signals 96 in Figure 1B.

In this regard, the radar detection device 90 may include a transmitter for producing electromagnetic waves, and an antenna (either external or internal to the radar detecting device) for emitting the waves. Alternatively, the radar detection device 90 may emit a plurality of pulses. In some examples, the radar detection device 90 may comprise a receiving antenna, which may be embodied within the same or different physical structure as the antenna for emitting the radar signal. The radar detection device 90 may further include a receiver for converting a received radar signal into a format or data that may be processed by apparatus 25. In this regard, the apparatus 25 may process the detected radar signals to perform the operations described herein according to example embodiments. Additionally or alternatively, the detected radar signals may be converted by the radar detection device 90, and/or any other apparatus, to data that is processed by apparatus 25 to perform the operation described herein. In some examples, the radar detection device 90 may include a processor (e.g., processor 20), for processing the data converted by the receiver. In some examples, the processor of the radar detection device 90 may be implemented separately from processor 20.

Radar signals may be emitted from the radar detection device 90 in rapid succession. Detected radar signals received by the radar detecting device may indicate a detected object, such as the bed, other reference points, and/or individuals in the bed. The reflected radar signals may therefore be used to determine, monitor, and/or analyze the position of a body profile relative to the reference point. The data used to define a body profile, and to monitor the activity of an individual associated with a body profile is described in further detail hereinafter. In general, changes in frequency, intensity, and variation of the detected radio waves may further indicate movement of the body profiles, whether the movement includes a repositioning of the body, or a subtle movement such as that caused by breathing, a pulse, and/or the like.

Radar detection device 90 may be embodied within a same device or housing as apparatus 25. In some examples, radar detection device 90 may be configured to provide data to apparatus 25, such as over a network or by direct wired connection. As yet another example, the radar detection device 90 may provide data to a server or other memory device, and the apparatus 25 may access the data for subsequent processing as described herein and according to example embodiments.

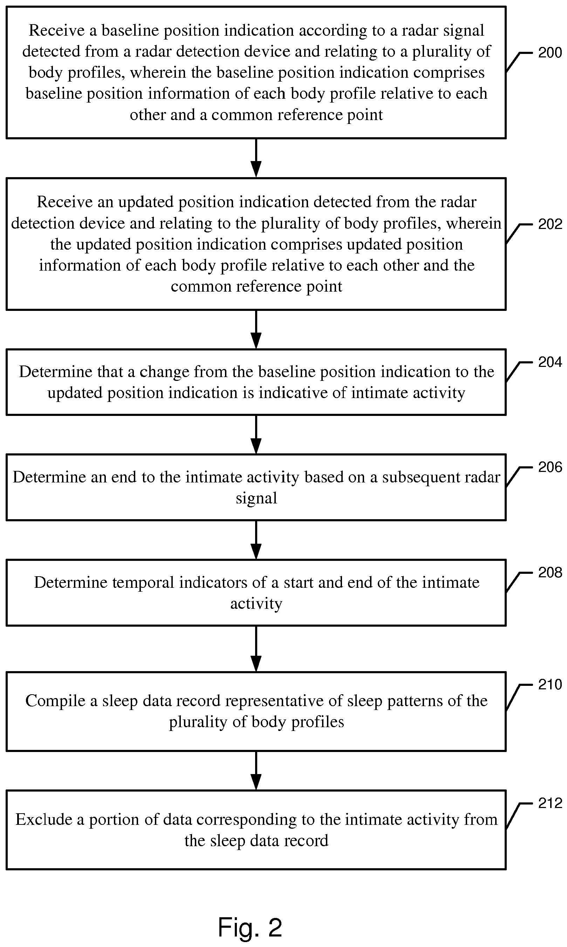

Referring now to Figure 2, the operations for determining an intimate activity has occurred or is occurring based on data collected with a radar detection device 90 are outlined in accordance with an example embodiment. In this regard and as described below, the operations of Figure 2 may be performed by apparatus 25.

As shown by operation 200, the apparatus 25 may include means, such as the processor 20, communication interface 24, the memory device 26, or the like, for receiving a baseline position indication according to a radar signal detected from a radar detection device 90 and relating to a plurality of body profiles, wherein the baseline position indication comprises baseline position information of each body profile relative to each other and a common reference point. In some examples, the baseline position indication may be generated by apparatus 25 according to the detected signal.

In this regard, the baseline position indication may be determined by the radar detection device 90 and/or apparatus 25 based on radar signals emitted and reflected in an instance in which an intimate activity is not occurring. For example, the radar detection device 90 and/or apparatus 25 may analyze signals received by the radar detection device 90 after any number of individuals (e.g., animals or people) enter the bed or are detected in the proximity of the reference point. The body profiles may therefore refer to any indication or data associated with an individual or body in the bed or in proximity to the reference point. The body profile may therefore include any data describing a body, body part, torso, limb and/or extremity. The body profile may further include any type of profile or data detectable by a radar including a heart rate signature, a breathing signature, a height, length, and/or the like. It will be appreciated that any reference to the term body made herein may be attributed to any data from the body profile.

The reference point is described as common to the two or more body profiles due to the two or more bodies lying on the same bed, or being detected within the vicinity of the same reference point. As described above, the common reference point may be any point in space, or detected planar surface, such as the top of the mattress. The apparatus 25 and/or radar detection device 90 may determine the reference point as any stable or near-stable object detected by the radar. For example, the common reference point may reflect consistent radar signals in various moments of time or throughout extended periods of time. In some examples, the common reference point may be identified according to the radar signals, when it is determined that no people or other participants are in the vicinity of the reference point (e.g., before bedtime).

In some examples, the radar detection system and/or apparatus 25 may differentiate between the reference point and the body profiles based on the strength of the detected radar signals. The material or composition of the reference point may vary from characteristics of bodies such that the radar detection system or apparatus 25 may distinguish between the reference point and body profiles. For example, a mattress with coils or other internal metallic structure may reflect a higher intensity signal than that of a human body. Additionally, since the reference point may be detected prior to bodies entering the vicinity, the baseline information relating to the reference point may therefore be utilized.

As another example, apparatus 25 may process the data based on the detected radar signal to determine that when one individual gets on the bed, an additional reflectance is provided compared to the reflectance of the reference point. The apparatus 25 may therefore detect a second additional reflectance associated with a second body profile. In some examples, when the two people are sleeping, there may be a measurable distance (e.g., at least a threshold distance) between the two body profile reflectances, indicating that the distance between the two is consistent. In some examples, any such indications detected in the data according to the radar signals may be interpreted by apparatus 25 as baseline position information.

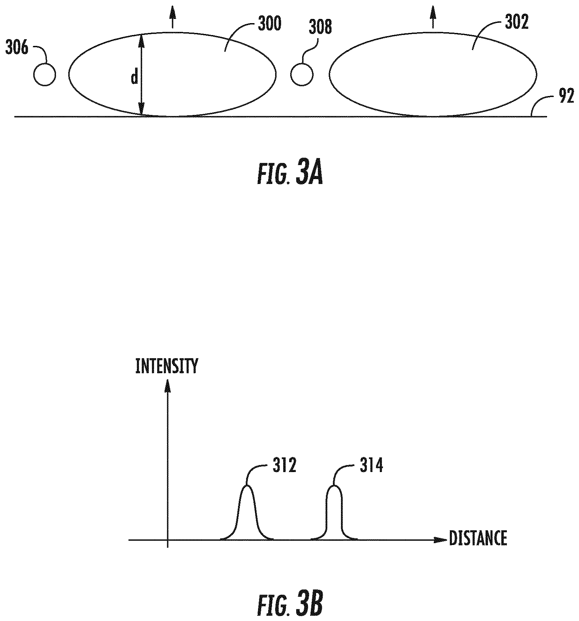

The baseline positon information may be determined, for example, by detecting two objects (e.g., people) laying side by side in a bed or relative to the reference point. See Figure 3A, which is an example cross-section view representative of a baseline position indication relating to two body profiles according to example embodiments. The cross-sections of two body profiles 300 and 302 detected by the radar detection device 90 are illustrated as lying on the reference point 92 (e.g., mattress). The torso of each individual has a measurable distance across, shown as "d" in the diagram. The smaller circles 306 and 308 represent cross sections through one person's arms. In some examples, such as that of Figure 3A, when two people are not undertaking intimate activities they may sleep or lie at a measurable distance apart. In some examples, there may be little or no sustained synchronisation of their movements and breathing patterns, which may be reflected by little or no variation (e.g., below a threshold variation) in the detected radar signals, and/or little or no changes in intensity (e.g., below a threshold intensity) of the radar signals. The baseline position information may therefore indicate that no intimate activities are occurring based on the measurable distance, intensity, and/or variation. Similarly, a baseline position indication may be determined based on that while one body profile reflects movement, a lack of synchronization with an individual associated with the other body profile may reflect a lack of intimate activity. The movement associated with only one body profile, or less than all of the observed body profiles, may be attributed to a restless state or poor sleep, for example.

Although the perspective of Figure 3A is from a substantially horizontal perspective, it will be appreciated that the radar detection device 90 may indeed be situated in any location, as long as the reference point and baseline position information of the body profiles can be detected by radar. For example, the radar detection device 90 may sit several centimeters higher than the reference point so as to detect signals received from both bodies. In some examples, the reference point is detected prior to any body profiles entering the vicinity, such that the two or more body profiles are detected thereafter based on a change in signal deflected from the reference point. The detected objects in the bed may be identified as body profiles based on characteristics consistent with a body. For example, an object having detected measurements falling within a predefined range, may be identified as a body.

The body profile may include any data detected by the radar detection system that describes the movement, positioning, or orientation of a body, person, animal and/or the like. For example, the body profile may include data describing the positioning of the torso or any body part, data reflecting the breath of an individual (e.g., movement or raising of the stomach and/or chest), movement of any body part reflecting a beating heart or pulse, and/or the like. In some examples, the body profile may reflect a body lying on its back, side or stomach, due to the measurements of the detected object relative to the reference point.

Figure 3B illustrates detected radar signals representative of the body profiles 300 and 302 detected from Figure 3A. The radar signals illustrated in Figure 3B may be those detected by the radar detection device, and received or accessed by apparatus 25. The radar signals illustrated in Figure 3B are provided on a plot showing radar signal intensity versus distance from the radar detection device 90. The two body profiles may appear as intensity peaks 312 and 314 at different distances from the radar detection device 90. Figure 3B therefore provides an example of baseline position information according to the detected radar signal.

In this regard, the baseline position information may include position information of each body profile relative to each other and the common reference point. For example, the baseline position information may indicate that both or each of the bodies associated with the respective body profiles are laying on top of the reference point (e.g., mattress). The baseline position information may therefore include a distance between the body profiles, which may be determined according to the detected distances of respective body profiles from the radar detection device 90. The distance between bodies may be measured as a distance between the edges of each of the body profiles. As another example, the positions of the body profiles relative to each other may include a distance of respective measured or detected centers of the body profiles. In this regard, the centers of the body profiles may be determined based on a center of a cross-section of the body profiles and/or a center of mass. The baseline position information indicated by the radar signal may also indicate information regarding movements or breathing patterns.

In some examples, the radar detection device 90 may be positioned such that the angles of the radar do not necessarily capture every contoured edge of a body. In this regard, the radar detection device and/or apparatus 25 may process the body profiles to estimate or determine the edge of the bodies and/or data otherwise describing the location of the body profiles relative to each other and/or the common reference point. For example, based upon the radar signal that is received, the edge of the far side of a body (relative to the radar detection device) may be estimated based upon symmetry with the edge of the near side of the body, which may be more readily interrogated by the radar detection device.

As shown by operation 202 in Figure 2, the apparatus 25 may include means, such as the processor 20, communication interface 24, the memory device 26, or the like, for receiving an updated position indication detected from the radar detection device 90 and relating to the plurality of body profiles, wherein the updated position indication comprises updated position information of each body profile relative to each other and the common reference point. In some examples, the apparatus 25 may generate the updated position information according to the detected signal.

In this regard, the radar detection device 90 may repeatedly emit and detect radar signals in the vicinity of the reference point. The apparatus 25 may therefore receive or access the data to determine that a change or update to the positioning information of any of the body profiles relative to each other or the common reference point has occurred.

As shown by operation 204, the apparatus 25 may include means, such as the processor 20, the memory device 26, or the like, for determining that a change from the baseline position indication to the updated position indication is indicative of intimate activity. Apparatus 25 may therefore calculate a change from the baseline position indication to the updated position indication by comparing or subtracting quantitative measurements such as the position of the respective body profiles relative to each other and/or the common reference point. The determination that the change is indicative of intimate activity may include an indication that the people, persons, animals, other individuals and/or the like associated with the body profiles are engaging in an intimate activity. The detected radar signals may therefore be continually or repeatedly monitored by apparatus 25 to identify any such changes and determine whether the change is indicative of an intimate activity. For example, the detected radar signals may be received, processed, and monitored at a regular time interval. The apparatus 25 may therefore access and process data regarding the positioning of the body profiles at various timestamps.

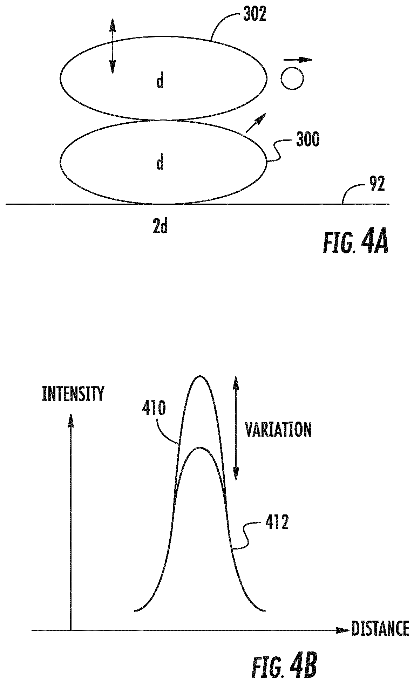

As illustrated in Figure 4A, two people may move into close contact with each other with one taking a higher position over the other, as illustrated by the body profiles 300 and 302. Apparatus 25 may compare the baseline position information to the updated position information to determine that the change is indicative of intimate activity. For example, the change based on the detected radar signals may reflect an increased rate and frequency of movements, some synchronisation of movements, elevated respiration rates, non-repetitive breaks in breathing (e.g. kissing) and/or periods of increased heart rates. The change may therefore be determined to exceed or meet a specific threshold associated with any of the aforementioned indications of intimate activity. For example, the thresholds required to be met to indicate an intimate activity may be predefined and stored on memory device 26.

As another example of determining that a change from the baseline position indication to the updated position indication is indicative of intimate activity, apparatus 25 may measure changes in body profiles as illustrated in plots of the body profile. As illustrated in Figure 4A, apparatus 25 may detect the close proximity of the two body profiles based on a total height of the body profiles indicated by the detected radar signal. For example, the radar signal may indicate that the associated bodies are positioned having a greater total height than one person with respect to the common reference point. This is shown in Figure 4A as "2d," as the total height of the detected body profiles may be approximately the sum of the separate body profile heights. In this regard, apparatus 25 may determine a change in at least a height or width of a cross-section of the plurality of body profiles is indicative of an intimate activity.

As illustrated in Figure 4A, apparatus 25 may access or receive updated position information as detected by the radar detection device 90. As reflected in the signals illustrated in Figure 4A, the detected radar signals may indicate the increase in size of the two people in close contact. For example, the body profiles may be determined to be an equal or near-equal distance from the radar detection device 90, or the distances may determine to be within a threshold distance of each other. In some examples, the change from a relatively larger distance of the body profiles from the radar detection device 90 may be compared to the updated position information indicating a relatively smaller distance of the bodies from the radar detection device 90. Apparatus 25 may therefore identify such a change in distance of at least one of the plurality of body profiles from the radar detection device and/or a change in distance of the plurality of body profiles from each other as indicative of an intimate activity.

In some examples, the intensity of the signal may change with movement of the two people. This variation is indicated in Figure 4B. The two body profiles may appear as intensity peaks 410 and 412, representing respective bodies positioned at different distances from the radar detection device 90. Radar detection works on the principle of emitting short bursts of radio energy that are transmitted and reflected off a target (e.g., a body profile) which is returned as an echo. The targets reflect a limited amount of radar energy. In Figure 4B, when the two objects (e.g., bodies) are in close physical proximity, the distance between them is reduced and there is a variation in the reflection as a result of change in physical cross sectional area of the object as indicated in the detected radar signals. Further, the peak of the plotted radar signal intensity may broaden or shrink as a function of time. Apparatus 25 may determine that any such variation of the intensity peaks over time is indicative of intimate activity. For example, changes in positioning indicating a pattern of back and forth movement may be processed and identified by apparatus 25 as being attributed to intimate activity.

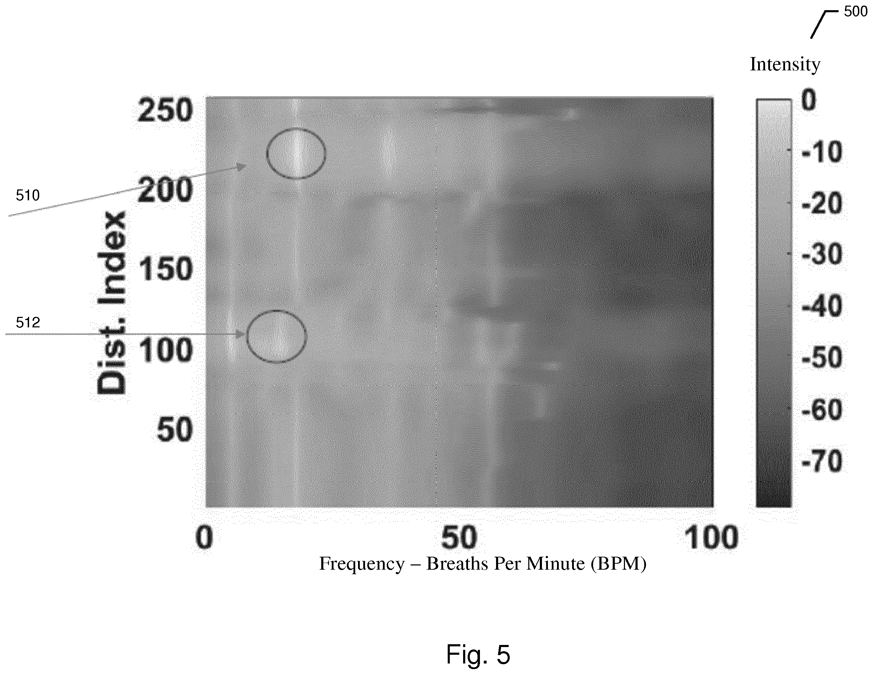

Figure 5 illustrates a diagram on which the detected radar signals may be charted, to indicate the distance from body profiles to the radar detection device 90 (e.g., Dist. Index), the frequency (e.g., breaths per minute or BPM) of the signals as a function of time, and the intensity of the signals. The intensity of the associated radar signal is indicated by the color in the chart. As indicated in the intensity color scale 500, the lighter colors starting from the top of the scale indicate a relatively higher intensity while the darker colors appearing toward the bottom of the scale indicate a relatively lower intensity.

Areas 510 and 512 of the chart having concentrated areas of lighter coloring may therefore be indicative of two respective body profiles, for example, due to the concentrated higher intensities relative to the other radar signals on the chart. Area 510 may indicate a body profile having an associated body positioned approximately 1.2 meters from the radar detection device and reflecting a respiration rate of 22 breaths per minute. Area 512 may indicate a body profile having an associated body positioned approximately 0.6 meters from the radar detection device and reflecting a respiration rate of 15 breaths per minute.

As another example, the apparatus 25 may measure the frequency of a detected radar signal, and detect movement, breathing and heart rate to be distinguished within the same signal. The changes in frequency are known as the Doppler effect. Apparatus 25 may further analyse the frequency of the detected radar signals to determine that the signal is indicative of an intimate activity. For example, a threshold frequency may be defined, such that if the frequency of the detected radar signal meets, exceeds or falls below the threshold frequency, the apparatus 25 determines an intimate activity has occurred or is occurring.

Several examples of changes identified by apparatus 25 in the detected radar signal as indicative of an intimate activity are provided herein. It will be appreciated that any combination of measurements or thresholds as described herein may be used to determine whether an intimate activity is reflected in the radar signal. For example, any data relating to the distance (of body profiles from each other, from the radar detection device 90, and/or from the reference point), variation, intensity, and/or frequency may be considered in determining whether or not intimate activity has occurred. For instance, apparatus 25, such as with processor 20, may apply weights to any of the aforementioned measurements such that the detected radar signal is scored. The score may indicate, for example, a likelihood of an intimate activity having occurred in the associated data.

Additionally or alternatively, any of the changes reflected in radar signals may be determined to be attributed to intimate activity when they occur within a specified order or within a certain time period of each other. For example, initially, the body profiles may be detected to move closer together, and after one to two minutes, then additional characteristics such as heavy breathing or synchronized movement may be identified based on the radar signals. In such an example, apparatus 25 may determine occurrence of an intimate activity based on the sequence of changes in the detected radar signal.

Accordingly, apparatus 25 may be configured to discriminate between different types of activity in bed, particular those attributed to sleep and those activities not attributed to sleep.

Continuing with the operations of Figure 2, as shown by operation 206, the apparatus 25 may include means, such as the processor 20, the memory device 26, or the like, for determining an end to the intimate activity based on a subsequent radar signal. In this regard, apparatus 25 may continue to monitor radar signals detected by the radar detection device 90, and process the data as described above, or similarly to as described above as with respect to operations 200, 202, and/or 204. Apparatus 25 may process the subsequent radar signal to determine an end to the intimate activity has occurred. For example, a change from the updated position information indicating a drop or decline in any of the measured indicators by a threshold amount may be indicative of the end to the intimate activity. As another example, a subsequent radar signal having similar (e.g., within a threshold difference of) characteristics or measurements to that of the baseline positon indication may be indicative of an end to the intimate activity. For example, when the subsequent radar signal indicates that two body profiles are positioned further apart than during the intimate activity, the apparatus 25 may determine an end to the intimate activity. As another example, apparatus 25 may determine an end to intimate activity when body profiles have returned to positions such that the cross-section width or height of the body profiles are consistent with bodies lying separately instead of together.

As such, as shown by operation 208, the apparatus 25 may include means, such as the processor 20, the memory device 26, or the like, for determining temporal indicators of a start and end of the intimate activity. The temporal indicators may be determined relative to the detected radar signal data and associated with the various signals or data representative thereof, such as on memory device 26. As another example, the temporal indicators may be timestamps indicating a point in time at which a particular radar signal was detected and which apparatus 25 determined to be indicative of the start and/or end to the intimate activity. The timestamps may be stored independently of the associated radar signal data, but may for instance be matched with the timestamps of the radar signal data subsequently, or otherwise applied so that data corresponding to the intimate activity may be distinguished from data that does not correspond to the intimate activity (e.g., data acquired from radar signals detected during sleep).

As shown by operation 210, the apparatus 25 may include means, such as the processor 20, the memory device 26, or the like, for compiling a sleep data record representative of sleep patterns associated with the plurality of body profiles. The apparatus 25 may therefore convert the detected radar signals, such as those detected by radar detection device 90, to a sleep data record. For example, the sleep data record may comprise data captured and extracted from radar signals detected over an 8 hour span during which a user enters the bed or the vicinity of the common reference point. The apparatus 25 may compile the sleep data associated with one or more of the plurality of body profiles. In some examples, apparatus 25 may store the sleep data on memory device 26 in association with an identifier of an individual associated with a body profile. In some examples, the apparatus 25 may compile the sleep data record prior to performing any of the operations 200, 202, 206, 208 and/or 210 described above.

As shown by operation 212, the apparatus 25 may include means, such as the processor 20, the memory device 26, or the like, for excluding a portion of data corresponding to the intimate activity from the sleep data record. The apparatus 25, such as with processor 20, may for example utilize the temporal indicators of the intimate activity, and delete the corresponding data from the memory device 26. As another example, the apparatus may indicate on the memory device 26 that particular data records are associated with the determined intimate activity such that the corresponding data may not be retrieved or accessed by other devices or services. Additionally or alternatively, apparatus 25 may associate the data corresponding to the intimate activity with a particular security requirement, such that access to the data is only allowed by authorized users or services. In some examples, apparatus 25 may determine or perform algorithms to assign likely labels (e.g., metadata) to the data corresponding to the intimate activity.

In some embodiments, the apparatus 25 may include means, such as the processor 20, user interface 22, and/or the like, for prompting a user to confirm exclusion of the data corresponding to an intimate activity from a sleep data record. In this regard, the user interface 22 may alert the user that an intimate activity is detected, and the user (e.g., the sleep study participant, or individual associated with any of the plurality of body profiles) may indicate or direct the apparatus 25 to exclude the data associated with the occurrence of intimate activity from the sleep data record and/or obscure the data associated with the occurrence of intimate activity.

In some examples, apparatus 25 may provide an option of applying different privacy functions to the sleep data record. For example, the apparatus 25 may enable the user to delete all the data, or label the data with metadata to indicate that the person was awake but no recording of activity, awake but activity indicated without labeling the activity specifically, or any combination thereof, for example. In some cases, the user may choose to leave the data in the sleep data record. In this regard, the user may prevent private data from being exposed, may provide the data for analysis by the system, or any combination thereof. Example embodiments may therefore provide privacy regarding the intimate activity while still allowing other portions of the sleep data record to be transmitted to other systems, services, and/or users. As such, the method and apparatus 25 of an example embodiment may provide technical advantages relating to efficiently, accurately and timely identifying instances of intimate activity such that the corresponding data may be maintained privately.

As described above, Figure 2 illustrates a flowchart of an apparatus 25, method, and computer program product according to example embodiments of the disclosure. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device 26 of an apparatus 25 employing an embodiment of the present disclosure and executed by a processor 20 of the apparatus 25. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

Accordingly, blocks of the flowchart support combinations of means for performing the specified functions and combinations of operations for performing the specified functions for performing the specified functions. It will also be understood that one or more blocks of the flowchart, and combinations of blocks in the flowchart, may be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included. Modifications, additions, or amplifications to the operations above may be performed in any order and in any combination.

Many modifications and other embodiments of the disclosures set forth herein will come to mind to one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A method comprising:
receiving (200) a baseline position indication according to a radar signal detected from a radar detection device and relating to a plurality of body profiles, wherein the baseline position indication comprises baseline position information of each body profile relative to each other and a common reference point;
receiving (202) an updated position indication detected from the radar detection device and relating to the plurality of body profiles, wherein the updated position indication comprises updated position information of each body profile relative to each other and the common reference point;
determining (204), with a processor, that a change from the baseline position indication to the updated position indication is indicative of intimate activity, wherein intimate activity is any physical activity occurring between at least two individuals associated with said body profiles that is not attributed to sleep;
**characterised in that** the method further comprises: compiling (210) a sleep data record representative of sleep patterns associated with the plurality of body profiles; and
excluding (212) a portion of data corresponding to the intimate activity from the sleep data record.

2. The method according to claim 1, further comprising:
determining (206) an end to the intimate activity based on a subsequent radar signal.

3. The method according to any of claims 1-2, further comprising:
determining (208) temporal indicators of a start and end of the intimate activity.

4. The method according to any of claims 1-3, wherein the portion of data corresponding to the intimate activity is excluded from the sleep data record in response to a user input.

5. The method according to any of claims 1-4, wherein the change indicative of the plurality of body profiles engaging in intimate activity is based on at least a change in distance of at least one of the plurality of body profiles from the radar detection device.

6. The method according to any of claims 1-5, wherein the change indicative of the plurality of body profiles engaging in intimate activity is based on a change in at least a height or width of a cross-section of the plurality of body profiles.

7. The method according to any of claims 1-6, wherein the change indicative of the plurality of body profiles engaging in intimate activity is based on a variation of the detected radar signal as a function of time.

8. The method according to any of claims 1-7, wherein the change indicative of the plurality of body profiles engaging in intimate activity is based on an intensity of the detected radar signal.

9. The method according to any of claims 1-8, wherein the change indicative of the plurality of body profiles engaging in intimate activity is based on a frequency of the detected radar signal.

10. The method according to any of claims 1-9, wherein the change indicative of the plurality of body profiles engaging in intimate activity is based on a change in distance of the plurality of body profiles from each other.

11. An apparatus comprising at least one radar signal detection device, at least one processor,
and at least one memory including computer program code, the at least one memory and the computer program code configured to, with the processor, cause the apparatus to at least:
generate a baseline position indication according to a signal detected from the signal detection device and relating to a plurality of body profiles, wherein the baseline position indication comprises baseline position information of each body profile relative to each other and a common reference point;
generate an updated position indication according to the signal detected from the signal detection device and relating to the plurality of body profiles, wherein the updated position indication comprises updated position information of each body profile relative to each other and the common reference point;
determine that a change from the baseline position indication to the updated position indication is indicative of the plurality of body profiles engaging in intimate activity, wherein intimate activity is any physical activity occurring between at least two individuals associated with said body profiles
that is not attributed to sleep; **characterised in that** the apparatus is further caused to: compile a sleep data record representative of sleep patterns associated with the plurality of body profiles; and
exclude a portion of data corresponding to the intimate activity from the sleep data record.

12. The apparatus according to claim 11, wherein the signal detection device comprises a radar detection device configured to:
emit a radar signal with a transmitter; and
with a transceiver, detect the radar signal reflected from the plurality of body profiles and the common reference point, wherein the baseline position indication and the updated position indication are generated according to the reflected radar signal.

13. A computer program product comprising at least one non-transitory computer-readable storage medium having computer-executable program code instructions stored therein, the computer-executable program code instructions comprising program code instructions which, when executed by a computer, cause the computer to at least:
receive (200) a baseline position indication according to a radar signal detected from a radar detection device and relating to a plurality of body profiles, wherein the baseline position indication comprises baseline position information of each body profile relative to each other and a common reference point;
receive (202) an updated position indication detected from the radar detection device and relating to the plurality of body profiles, wherein the updated position indication comprises updated position information of each body profile relative to each other and the common reference point;
determine (204) that a change from the baseline position indication to the updated position indication is indicative of the plurality of body profiles engaging in intimate activity, wherein intimate activity is any physical activity occurring between at least two individuals associated with said body profiles that is not attributed to sleep;
**characterised in that** the instructions further: compile (210) a sleep data record representative of sleep patterns associated with the plurality of body profiles; and
exclude (212) a portion of data corresponding to the intimate activity from the sleep data record.

14. The computer program product according to claim 13, wherein the computer-executable program code instructions further comprise program code instructions which, when executed by a computer, cause the computer to perform the method of any of claims 2-10.

## Patentansprüche

1. Verfahren, das Folgendes umfasst:
Empfangen (200) einer Basislinienpositionsanzeige gemäß einem Radarsignal, das von einer Radardetektionsvorrichtung detektiert wird und eine Vielzahl von Körperprofilen betrifft, wobei die Basislinienpositionsanzeige Basislinienpositionsinformationen von jedem Körperprofil relativ zueinander und einen gemeinsamen Referenzpunkt umfasst;
Empfangen (202) einer aktualisierten Positionsanzeige, die von der Radardetektionsvorrichtung detektiert wird und die Vielzahl von Körperprofilen betrifft, wobei die aktualisierte Positionsanzeige aktualisierte Positionsinformationen von jedem Körperprofil relativ zueinander und den gemeinsamen Referenzpunkt umfasst;
Bestimmen (204) mit einem Prozessor, dass eine Änderung von der Basislinienpositionsanzeige zur aktualisierten Positionsanzeige eine intime Aktivität anzeigt, wobei eine intime Aktivität eine körperliche Aktivität ist, die zwischen mindestens zwei Individuen, die mit den Körperprofilen verknüpft sind, erfolgt und nicht Schlaf zugeschrieben wird;
**dadurch gekennzeichnet, dass** das Verfahren ferner Folgendes umfasst:
Kompilieren (210) eines Schlafdatensatzes, der für Schlafmuster repräsentativ ist, die mit der Vielzahl von Körperprofilen verknüpft sind; und
Ausschließen (212) eines Datenabschnitts, der der intimen Aktivität entspricht, aus dem Schlafdatensatz.

2. Verfahren nach Anspruch 1, das ferner Folgendes umfasst:
Bestimmen (206) eines Endes der intimen Aktivität auf Basis eines nachfolgenden Radarsignals.

3. Verfahren nach einem der Ansprüche 1-2, das ferner Folgendes umfasst:
Bestimmen (208) von zeitlichen Indikatoren eines Beginns und eines Endes der intimen Aktivität.

4. Verfahren nach einem der Ansprüche 1-3, wobei der Datenabschnitt, der der intimen Aktivität entspricht, in Reaktion auf eine Benutzereingabe aus dem Schlafdatensatz ausgeschlossen wird.

5. Verfahren nach einem der Ansprüche 1-4, wobei die Änderung, die die Vielzahl von Körperprofilen anzeigt, die an einer intimen Aktivität beteiligt sind, mindestens auf einer Änderung eines Abstands von mindestens einem der Vielzahl von Körperprofilen von der Radardetektionsvorrichtung basiert.

6. Verfahren nach einem der Ansprüche 1-5, wobei die Änderung, die die Vielzahl von Körperprofilen anzeigt, die an einer intimen Aktivität beteiligt sind, auf einer Änderung von mindestens einer Höhe oder einer Breite eines Querschnitts der Vielzahl von Körperprofilen basiert.

7. Verfahren nach einem der Ansprüche 1-6, wobei die Änderung, die die Vielzahl von Körperprofilen anzeigt, die an einer intimen Aktivität beteiligt sind, auf einer Variation des detektierten Radarsignals als einer Funktion der Zeit basiert.

8. Verfahren nach einem der Ansprüche 1-7, wobei die Änderung, die die Vielzahl von Körperprofilen anzeigt, die an einer intimen Aktivität beteiligt sind, auf einer Intensität des detektierten Radarsignals basiert.

9. Verfahren nach einem der Ansprüche 1-8, wobei die Änderung, die die Vielzahl von Körperprofilen anzeigt, die an einer intimen Aktivität beteiligt sind, auf einer Frequenz des detektierten Radarsignals basiert.

10. Verfahren nach einem der Ansprüche 1-9, wobei die Änderung, die die Vielzahl von Körperprofilen anzeigt, die an einer intimen Aktivität beteiligt sind, auf einer Änderung eines Abstands der Vielzahl von Körperprofilen voneinander basiert.

11. Einrichtung, die mindestens eine Radarsignaldetektionsvorrichtung, mindestens einen Prozessor und mindestens einen Speicher, der Computerprogrammcode beinhaltet, umfasst, wobei der mindestens eine Speicher und der Computerprogrammcode dazu ausgelegt sind, mit dem Prozessor zu bewirken, dass die Einrichtung mindestens Folgendes durchführt:
Erzeugen einer Basislinienpositionsanzeige gemäß einem Signal, das von der Signaldetektionsvorrichtung detektiert wird und eine Vielzahl von Körperprofilen betrifft, wobei die Basislinienpositionsanzeige Basislinienpositionsinformationen von jedem Körperprofil relativ zueinander und einen gemeinsamen Referenzpunkt umfasst;
Erzeugen einer aktualisierten Positionsanzeige gemäß dem Signal, das von der Signaldetektionsvorrichtung detektiert wird und die Vielzahl von Körperprofilen betrifft, wobei die aktualisierte Positionsanzeige aktualisierte Positionsinformationen von jedem Körperprofil relativ zueinander und den gemeinsamen Referenzpunkt umfasst;
Bestimmen, dass eine Änderung von der Basislinienpositionsanzeige zur aktualisierten Positionsanzeige die Vielzahl von Körperprofilen, die an einer intimen Aktivität beteiligt sind, anzeigt, wobei eine intime Aktivität eine körperliche Aktivität ist, die zwischen mindestens zwei Individuen, die mit den Körperprofilen verknüpft sind, erfolgt und nicht Schlaf zugeschrieben wird;
**dadurch gekennzeichnet, dass** bewirkt wird, dass die Vorrichtung ferner Folgendes durchführt:
Kompilieren eines Schlafdatensatzes, der für Schlafmuster repräsentativ ist, die mit der Vielzahl von Körperprofilen verknüpft sind; und
Ausschließen eines Datenabschnitts, der der intimen Aktivität entspricht, aus dem Schlafdatensatz.

12. Einrichtung nach Anspruch 11, wobei die Signaldetektionsvorrichtung eine Radardetektionsvorrichtung umfasst, die zu Folgendem ausgelegt ist:
Emittieren eines Radarsignals mit einem Sender; und
Detektieren des Radarsignals, das von der Vielzahl von Körperprofilen reflektiert wird, und des gemeinsamen Referenzpunkts mit einem Sendeempfänger, wobei die Basislinienpositionsanzeige und die aktualisierte Positionsanzeige gemäß dem reflektierten Radarsignal erzeugt werden.

13. Computerprogrammprodukt, das mindestens ein nichttransitorisches computerlesbares Speichermedium umfasst, in dem computerausführbare Programmcodeanweisungen gespeichert sind, wobei die computerausführbaren Programmcodeanweisungen Programmcodeanweisungen umfassen, die, wenn sie von einem Computer ausgeführt werden, bewirken, dass der Computer mindestens Folgendes durchführt:
Empfangen (200) einer Basislinienpositionsanzeige gemäß einem Radarsignal, das von einer Radardetektionsvorrichtung detektiert wird und eine Vielzahl von Körperprofilen betrifft, wobei die Basislinienpositionsanzeige Basislinienpositionsinformationen von jedem Körperprofil relativ zueinander und einen gemeinsamen Referenzpunkt umfasst;
Empfangen (202) einer aktualisierten Positionsanzeige, die von der Radardetektionsvorrichtung detektiert wird und die Vielzahl von Körperprofilen betrifft, wobei die aktualisierte Positionsanzeige aktualisierte Positionsinformationen von jedem Körperprofil relativ zueinander und den gemeinsamen Referenzpunkt umfasst;
Bestimmen (204), dass eine Änderung von der Basislinienpositionsanzeige zur aktualisierten Positionsanzeige die Vielzahl von Körperprofilen, die an einer intimen Aktivität beteiligt sind, anzeigt, wobei eine intime Aktivität eine körperliche Aktivität ist, die zwischen mindestens zwei Individuen, die mit den Körperprofilen verknüpft sind, erfolgt und nicht Schlaf zugeschrieben wird;
**dadurch gekennzeichnet, dass** die Anweisungen ferner:
einen Schlafdatensatz kompilieren (210), der für Schlafmuster repräsentativ ist, die mit der Vielzahl von Körperprofilen verknüpft sind; und
einen Datenabschnitt, der der intimen Aktivität entspricht, aus dem Schlafdatensatz ausschließen (212).

14. Computerprogrammprodukt nach Anspruch 13, wobei die computerausführbaren Programmcodeanweisungen ferner Programmcodeanweisungen umfassen, die, wenn sie von einem Computer ausgeführt werden, bewirken, dass der Computer das Verfahren von einem der Ansprüche 2-10 durchführt.

## Revendications

1. Procédé comprenant les étapes consistant à :
recevoir (200) une indication de position de ligne de base selon un signal radar détecté à partir d'un dispositif de détection radar et concernant une pluralité de profils corporels, dans lequel l'indication de position de ligne de base comprend des informations de position de ligne de base de chaque profil corporel les uns par rapport aux autres et par rapport à un point de référence commun ;
recevoir (202) une indication de position mise à jour détectée à partir du dispositif de détection radar et concernant la pluralité de profils corporels, dans lequel l'indication de position mise à jour comprend des informations de position mises à jour de chaque profil corporel les uns par rapport aux autres et par rapport au point de référence commun ;
déterminer (204), à l'aide d'un processeur, qu'un changement de l'indication de position de ligne de base à l'indication de position mise à jour est indicatif d'une activité intime, dans lequel une activité intime est toute activité physique se produisant entre au moins deux individus associés auxdits profils corporels qui n'est pas attribuée au sommeil ;
**caractérisé en ce que** le procédé comprend en outre les étapes consistant à :
compiler (210) un enregistrement de données de sommeil représentatif de phases du sommeil associées à la pluralité de profils corporels ; et
exclure (212) une partie de données correspondant à l'activité intime de l'enregistrement de données de sommeil.

2. Procédé selon la revendication 1, comprenant en outre l'étape consistant à :
déterminer (206) une fin de l'activité intime sur la base d'un signal radar ultérieur.

3. Procédé selon l'une quelconque des revendications 1 et 2, comprenant en outre l'étape consistant à :
déterminer (208) des indicateurs temporels d'un début et d'une fin de l'activité intime.

4. Procédé selon l'une quelconque des revendications 1 à 3, dans lequel la partie de données correspondant à l'activité intime est exclue de l'enregistrement de données de sommeil en réponse à une entrée utilisateur.

5. Procédé selon l'une quelconque des revendications 1 à 4, dans lequel le changement indiquant que la pluralité de profils corporels se livrent à une activité intime est basé sur au moins un changement de distance d'au moins un de la pluralité de profils corporels par rapport au dispositif de détection radar.

6. Procédé selon l'une quelconque des revendications 1 à 5, dans lequel le changement indiquant que la pluralité de profils corporels se livrent à une activité intime est basé sur un changement d'au moins une hauteur ou une largeur d'une section transversale de la pluralité de profils corporels.

7. Procédé selon l'une quelconque des revendications 1 à 6, dans lequel le changement indiquant que la pluralité de profils corporels se livrent à une activité intime est basé sur une variation du signal radar détecté en fonction du temps.

8. Procédé selon l'une quelconque des revendications 1 à 7, dans lequel le changement indiquant que la pluralité de profils corporels se livrent à une activité intime est basé sur une intensité du signal radar détecté.

9. Procédé selon l'une quelconque des revendications 1 à 8, dans lequel le changement indiquant que la pluralité de profils corporels se livrent à une activité intime est basé sur une fréquence du signal radar détecté.

10. Procédé selon l'une quelconque des revendications 1 à 9, dans lequel le changement indiquant que la pluralité de profils corporels se livrent à une activité intime est basé sur un changement de distance de la pluralité de profils corporels les uns par rapport aux autres.

11. Appareil comprenant au moins un dispositif de détection de signal radar, au moins un processeur, et au moins une mémoire comportant un code de programme informatique, l'au moins une mémoire et le code de programme informatique étant configurés pour, avec le processeur, amener l'appareil à au moins :
générer une indication de position de ligne de base selon un signal détecté à partir du dispositif de détection de signal et concernant une pluralité de profils corporels, dans lequel l'indication de position de ligne de base comprend des informations de position de ligne de base de chaque profil corporel les uns par rapport aux autres et par rapport à un point de référence commun ;
générer une indication de position mise à jour selon le signal détecté à partir du dispositif de détection de signal et concernant la pluralité de profils corporels, dans lequel l'indication de position mise à jour comprend des informations de position mises à jour de chaque profil corporel les uns par rapport aux autres et par rapport au point de référence commun ;
déterminer qu'un changement de l'indication de position de ligne de base à l'indication de position mise à jour indique que la pluralité de profils corporels se livrent à une activité intime, dans lequel une activité intime est toute activité physique se produisant entre au moins deux individus associés auxdits profils corporels qui n'est pas attribuée au sommeil ;
**caractérisé en ce que** l'appareil est en outre amené à :
compiler un enregistrement de données de sommeil représentatif des phases du sommeil associées à la pluralité de profils corporels ; et
exclure une partie de données correspondant à l'activité intime de l'enregistrement de données de sommeil.

12. Appareil selon la revendication 11, dans lequel le dispositif de détection de signal comprend un dispositif de détection radar configuré pour :
émettre un signal radar à l'aide d'un émetteur ; et
à l'aide d'un émetteur-récepteur, détecter le signal radar réfléchi par la pluralité de profils corporels et le point de référence commun, dans lequel l'indication de position de ligne de base et l'indication de position mise à jour sont générées selon le signal radar réfléchi.

13. Produit de programme informatique comprenant au moins un support de stockage non transitoire lisible par ordinateur dans lequel sont stockées des instructions de code de programme exécutables par ordinateur, les instructions de code de programme exécutables par ordinateur comprenant des instructions de code de programme qui, lorsqu'elles sont exécutées par un ordinateur, amènent l'ordinateur à au moins :
recevoir (200) une indication de position de ligne de base selon un signal radar détecté à partir d'un dispositif de détection radar et concernant une pluralité de profils corporels, dans lequel l'indication de position de ligne de base comprend des informations de position de ligne de base de chaque profil corporel les uns par rapport aux autres et par rapport à un point de référence commun ;
recevoir (202) une indication de position mise à jour détectée à partir du dispositif de détection radar et concernant la pluralité de profils corporels, dans lequel l'indication de position mise à jour comprend des informations de position mises à jour de chaque profil corporel les uns par rapport aux autres et par rapport au point de référence commun ;
déterminer (204) qu'un changement de l'indication de position de ligne de base à l'indication de position mise à jour indique que la pluralité de profils corporels se livrent à une activité intime, dans lequel une activité intime est toute activité physique se produisant entre au moins deux individus associés auxdits profils corporels qui n'est pas attribuée au sommeil ;
**caractérisé en ce que** les instructions, en outre :
compilent (210) un enregistrement de données de sommeil représentatif de phases du sommeil associées à la pluralité de profils corporels ; et
excluent (212) une partie de données correspondant à l'activité intime de l'enregistrement de données de sommeil.

14. Produit de programme informatique selon la revendication 13, dans lequel les instructions de code de programme exécutables par ordinateur comprennent en outre des instructions de code de programme qui, lorsqu'elles sont exécutées par un ordinateur, amènent l'ordinateur à réaliser le procédé selon l'une quelconque des revendications 2 à 10.
